# EUROPÄISCHE PATENTSCHRIFT

(11) **EP 0 665 214 B1**
(45) Veröffentlichungstag und Bekanntmachung des Hinweises auf die Patenterteilung: **15.04.1998**
(21) Anmeldenummer: 95810037.2
(22) Anmeldetag: 19.01.1995
(51) Int. Cl.: C07C 235/54, C07C 235/32, C07C 235/50, C07C 233/38, C07C 233/36, C08G 59/44

(54) **Monocarbonsäureamide von Polyaminen**
Monocarboxylic acid amides of polyamines
Amides d'acides monocarboxyliques et de polyamines

(30) Priorität: 28.01.1994 CH 268/94
(43) Veröffentlichungstag der Anmeldung: 02.08.1995
(73) Patentinhaber: Ciba Specialty Chemicals Holding Inc., 4057 Basel (CH)
(72) Erfinder: Fischer, Dr. Walter, CH-4153 Reinach (CH); Helbling, Christine, CH-4127 Birsfelden (CH)

(56) Entgegenhaltungen:
- EP-A- 0 554 823
- CHEMICAL ABSTRACTS, Band 97, Nr. 25, 20. Dezember 1982, Columbus, Ohio, USA TAKEDA CHEMICAL INDUSTRIES "Polyamino compounds" Seite 810, Nr. 215 723b; & JP-A-57 091 963

## Beschreibung

Die vorliegende Erfindung betrifft neue Monocarbonsäureamide von bestimmten Di-und Triaminen, härtbare Epoxidharzgemische, enthaltend die neuen Monocarbonsäureamide, und die aus den härtbaren Epoxidharzgemischen durch Härtung erhaltenen Formstoffe, insbesondere Beschichtungen.

Zur Herstellung von chemikalienresistenten Beschichtungen auf Basis von Epoxidharzen werden als Härtungsmittel vor allem flüssige Polyamine eingesetzt, wie beispielsweise formulierte Gemische auf Basis von 4,4'-Diaminodiphenylmethan. Dieser Härter gilt aber als mutagen- und kanzerogenverdächtig und wird beispielsweise in USA seit Dezember 1992 mit aussergewöhnlichen Anwendungsauflagen seitens der staatlichen Behörden OSHA (Occupational Safety and Health Administration), Final Rule in the Federal Register on the Use of Methylene Dianiline, belegt. In ähnlicher Weise hat das schweizerische Bundesamt für Gesundheitswesen diesen Härter gemäss Giftliste 1 (Ausgabe 1991) in die Giftklasse 1* (kanzerogen) eingestuft. Es besteht somit ein Bedarf nach anderen aminischen Härtungsmitteln für Epoxidharzbeschichtungen.

Ein von der Firma Anchor Chemical unter der Bezeichnung Ancamine X2280 im Handel erhältliches flüssiges Polyamin auf Basis von polycyclischen Polyaminen eignet sich zur Herstellung von Epoxidharzbeschichtungen mit einer guten Chemikalienresistenz, mit Ausnahme gegenüber von Carbonsäuren, wie beispielsweise Essigsäure.

Es wurde nun gefunden, dass bestimmte neue Monocarbonsäureamide, die durch Umsetzung von bestimmten Hydroxymonocarbonsäuren oder deren Ester mit bestimmten Di- oder Triaminen erhalten werden, den Epoxidharzbeschichtungen eine bessere Chemikalienresistenz, inbesondere gegenüber Carbonsäuren, verleihen.

Gegenstand vorliegender Erfindung sind somit hydroxylgruppenhaltige Monocarbonsäureamide der Formel I 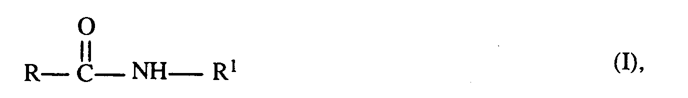 worin R für einen Rest der Formeln 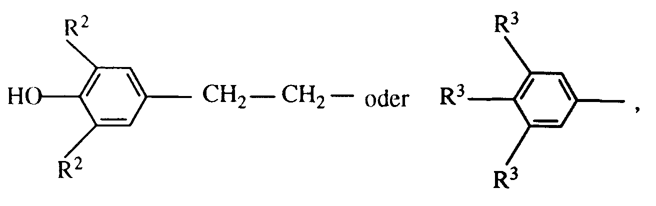 ein C₉-C₁₉-Alkyl, C₉-C₁₉-Alkenyl oder ein C₉-C₁₉-Alkdienyl steht, wobei jedes R² unabhängig voneinander je ein Wasserstoffatom oder ein C₁-C₄-Alkyl bedeutet, jedes R³ unabhängig voneinander je ein Wasserstoffatom oder eine Hydroxylgruppe bedeutet und mindestens ein R³ für eine Hydroxylgruppe steht, und R¹ für einen Rest der Formeln 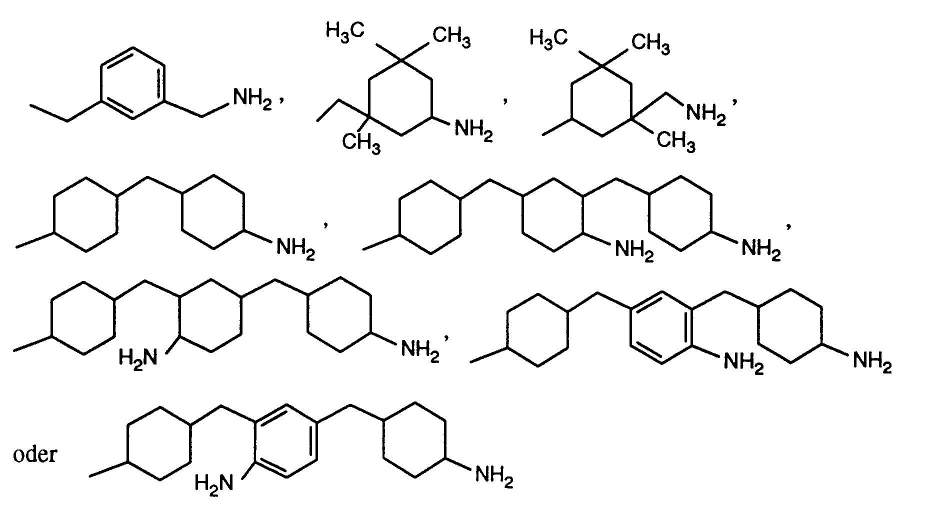 steht.

Von den erfindungsgemässen hydroxylgruppenhaltigen Monocarbonsäureamiden der Formel I sind solche bevorzugt, worin R für einen Rest der Formeln 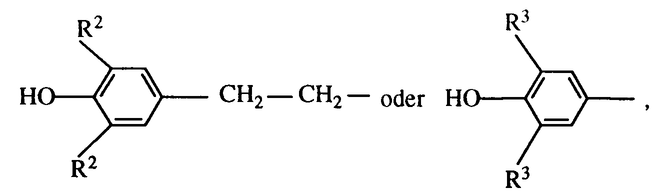 ein C₁₅-C₁₇-Alkyl oder C₁₅-C₁₇-Alkenyl steht, wobei jedes R² ein tert.-Butyl bedeutet, und jedes R³ je ein Wasserstoffatom oder je eine Hydroxylgruppe bedeutet.

Insbesondere bedeutet R in der Formel I den Rest der Formel

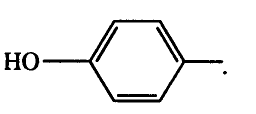

In den erfindungsgemässen Monocarbonsäureamiden bedeutet R¹ vorzugsweise einen Rest der Formeln

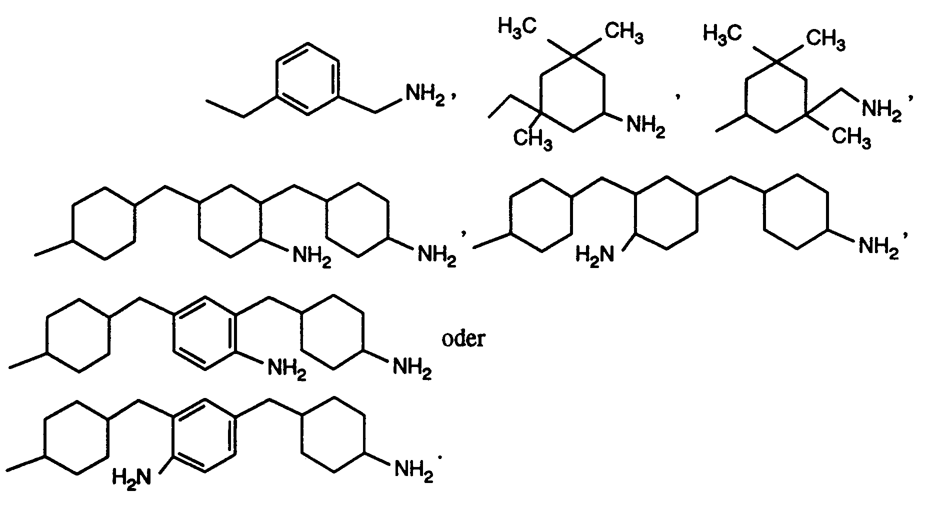

Wie eingangs erwähnt, stellen die erfindungsgemässen Verbindungen vorteilhafte aminische Härtungsmittel für Epoxidharze dar.

Gegenstand vorliegender Erfindung ist somit auch ein Härtungsmittel für Epoxidharze, das dadurch gekennzeichnet ist, dass es mindestens eine Verbindung der Formel I enthält.

Das erfindungsgemässe Härtungsmittel kann beispielsweise hergestellt werden, indem man eine Monocarbonsäure oder deren Ester der Formel II

R―COOR⁴ (II),

worin R die gleiche Bedeutung wie in Formel I hat und R⁴ ein Wasserstoffatom, C₁-C₆-Alkyl oder Phenyl bedeutet, mit einem Di- oder Triamin der Formel III

H₂N―R¹ (III),

worin R¹ die gleiche Bedeutung wie in Formel I hat, bei erhöhter Temperatur umsetzt, wobei man auf 1 Mol einer Verbindung der Formel II 2 bis 10 Mole einer Verbindung der Formel III einsetzt.

Im allgemeinen wird diese Umsetzung im Temperaturbereich von 80° bis 200°C vorgenommen. Vorzugsweise wendet man bei der Umsetzung der Monocarbonsäuren mit den Di- oder Triaminen Temperaturen von 160 bis 200°C und bei der Umsetzung der Monocarbonsäureester mit den Di- oder Triaminen Temperaturen von 100 bis 180°C an.

Vorzugsweise liegen bei der Umsetzung das Di- oder Triamin der Formel III in einem äquivalenten Überschuss vor, so dass bei der Umsetzung im wesentlichen die entsprechenden Diaminomonocarbonsäureamide und Triaminomonocarbonsäureamide neben einem Überschuss an Di- und Triamin entstehen. Für den Einsatz dieser Umsetzungsprodukte als Härtungsmittel ist es möglich, aber nicht erforderlich, die Umsetzungsprodukte von dem überschüssigen Di- oder Triamin zu befreien.

Die Umsetzung der Monocarbonsäuren bzw. deren Estern mit den Di- oder Triaminen kann auch in Gegenwart von Zusätzen, wie Lösungsmitteln, beispielsweise Butanol oder Xylol, Beschleunigern, beispielsweise Salicylsäure, Nonylphenol, Alkali-oder Erdalkaliperchlorate oder -nitrate, Flexibilisatoren, beispielsweise Polyethylenglykole, Fliessmittel, beispielsweise Benzylalkohol, Pigmenten, Füllstoffen oder Antioxidatien, durchgeführt werden.

Die Umsetzung kann auch gleichzeitig oder nacheinander mit verschiedenen Di-und/oder Triaminen und verschiedenen Monocarbonsäuren oder deren Estern durchgeführt werden, wobei die entsprechenden Produktegemische erhalten werden.

Die Monocarbonsäuren der Formel II und die Di- und Triamine der Formel III stellen bekannte Verbindungen dar und sind zum Teil im Handel erhältlich.

Wie eingangs erwähnt, können die erfindungsgemässen Monocarbonsäureamide zum Härten von üblichen Epoxidharzen eingesetzt werden.

Ein weiterer Gegenstand vorliegender Erfindung sind somit auch härtbare Gemische, enthaltend
(a) ein Epoxidharz mit mehr als einer 1,2-Epoxidgruppe im Molekül und
(b) mindestens eine erfindungsgemässe Verbindung der Formel I.

Als Epoxidharze (a), die in den erfindungsgemässen härtbaren Gemischen eingesetzt werden können, eignen sich die in der Epoxidharztechnik üblichen Epoxidharze. Beispiele für Epoxidharze sind:
I) Polyglycidyl- und Poly-(β-methylglycidyl)-ester, erhältlich durch Umsetzung einer Verbindung mit mindestens zwei Carboxylgruppen im Molekül und Epichlorhydrin bzw. β-Methylepichlorhydrin. Die Umsetzung erfolgt zweckmässig in der Gegenwart von Basen.
   Als Verbindung mit mindestens zwei Carboxylgruppen im Molekül können aliphatische Polycarbonsäuren verwendet werden. Beispiele für solche Polycarbonsäuren sind Oxalsäure, Bernsteinsäure, Glutarsäure, Adipinsäure, Pimelinsäure, Sebacinsäure, Korksäure, Azelainsäure oder dimerisierte bzw. trimerisierte Linolsäure.
   Es können aber auch cycloaliphatische Polycarbonsäuren eingesetzt werden, wie beispielsweise Tetrahydrophthalsäure, 4-Methyltetrahydrophthalsäure, Hexahydrophthalsäure oder 4-Methylhexahydrophthalsäure.
   Weiterhin können aromatische Polycarbonsäuren Verwendung finden, wie beispielsweise Phthalsäure, Isophthalsäure oder Terephthalsäure.
II) Polyglycidyl-oder Poly-(β-methylglycidyl)-ether, erhältlich durch Umsetzung einer Verbindung mit mindestens zwei freien alkoholischen Hydroxygruppen und/oder phenolischen Hydroxygruppen und Epichlorhydrin oder β-Methylepichlorhydrin unter alkalischen Bedingungen, oder in Anwesenheit eines sauren Katalysators und anschliessende Alkalibehandlung.
   Die Glycidylether dieses Typs leiten sich beispielsweise von acyclischen Alkoholen ab, wie von Ethylenglykol, Diethylenglykol und höheren Poly-(oxyethylen)-glykolen, Propan-1,2-diol oder Poly-(oxypropylen)-glykolen, Propan-1,3-diol, Butan-1,4-diol, Poly-(oxytetramethylen)-glykolen, Pentan-1,5-diol, Hexan-1,6-diol, Hexan-2,4,6-triol, Glycerin, 1,1,1-Trimethylolpropan, Pentaerythrit, Sorbit, sowie von Polyepichlorhydrinen. Sie leiten sich aber auch beispielsweise von cycloaliphatischen Alkoholen, wie 1,4-Cyclohexandimethanol, Bis-(4-hydroxycyclohexyl)-methan oder 2,2-Bis-(4-hydroxycyclohexyl)-propan, ab oder sie besitzen aromatische Kerne, wie N,N-Bis-(2-hydroxyethyl)-anilin oder p,p'-Bis-(2-hydroxyethylamino)-diphenylmethan.
   Die Glycidylether können sich auch von einkerningen Phenolen ableiten, wie beispielsweise von Resorcin oder Hydrochinon, oder sie basieren auf mehrkernigen Phenolen, wie beispielsweise Bis-(4-hydroxyphenyl)-methan, 4,4'-Dihydroxybiphenyl, Bis-(4-hydroxyphenyl)-sulfon, 1,1,2,2-Tetrakis-(4-hydroxyphenyl)-ethan, 2,2-Bis-(4-hydroxyphenyl)-propan, 2,2-Bis-(3,5-dibrom-4-hydroxyphenyl)-propan sowie von Novolaken, erhältlich durch Kondensation von Aldehyden, wie Formaldehyd, Acetaldehyd, Chloral oder Furfuraldehyd, mit Phenolen, wie Phenol, oder mit Phenolen, die im Kern mit Chloratomen oder C₁-C₉-Alkylgruppen substituiert sind, wie beispielsweise 4-Chlorphenol, 2-Methylphenol, oder 4-tert.-Butylphenol oder durch Kondensation mit Bisphenolen, wie solche der oben genannten Art.
III) Poly-(N-glycidyl)-verbindungen, erhältlich durch Dehydrochlorierung der Reaktionsprodukte von Epichlorhydrin mit Aminen, die mindestens zwei Aminwasserstoffatome enthalten. Bei diesen Aminen handelt es sich zum Beispiel um Anilin, n-Butylamin, Bis-(4-aminophenyl)-methan, m-Xylylendiamin oder Bis-(4-methylaminophenyl)-methan. Zu den Poly-(N-glycidyl)-verbindungen zählen aber auch Triglycidylisocyanurat, N,N'-Diglycidylderivate von Cycloalkylenharnstoffen, wie Ethylenharnstoff oder 1,3-Propylenharnstoff, und Diglycidylderivate von Hydantoinen, wie von 5,5-Dimethylhydantoin.
IV) Poly-(S-glycidyl)-verbindungen, beispielsweise Di-S-glycidylderivate, die sich von Dithiolen, wie beispielsweise Ethan-1,2-dithiol oder Bis-(4-mercaptomethylphenyl)-ether ableiten.
V) Cycloaliphatische Epoxidharze, beispielsweise Bis-(2,3-epoxycyclopentyl)-ether, 2,3-Epoxycyclopentylglycidylether, 1,2-Bis-(2,3-epoxycyclopentyloxy)-ethan oder 3,4-Epoxycyclohexylmethyl-3',4'-epoxycyclohexancarboxylat.

Es lassen sich aber auch Epoxidharze verwenden, bei denen die 1,2-Epoxidgruppen an unterschiedliche Heteroatome bzw. funktionelle Gruppen gebunden sind; zu diesen Verbindungen zählen beispielsweise das N,N,O-Triglycidylderivat des 4-Aminophenols, der Glycidylether-glycidylester der Salicylsäure, N-Glycidyl-N'-(2-glycidyloxypropyl)-5,5-dimethylhydantoin oder 2-Glycidyloxy-1,3-bis-(5,5-dimethyl-1-glycidylhydantoin-3-yl)-propan.

Bevorzugt verwendet man in den erfindungsgemässen härtbaren Gemischen als Epoxidharz (a) einen flüssigen oder viskosen Polyglycidylether oder -ester, insbesondere einen flüssigen oder viskosen Bisphenoldiglycidylether.

Die oben genannten Epoxidverbindungen sind bekannt und zum Teil im Handel erhältlich. Es können auch Gemische von Epoxidharzen verwendet werden.

Die Menge des eingesetzten Härtungsmittels richtet sich nach der chemischen Natur des Härtungsmittels und nach den gewünschten Eigenschaften der härtbaren Mischung und des gehärteten Produktes. Die maximale Menge kann leicht ermittelt werden. Normalerweise werden 0,75 bis 1,25 Äquivalente Aminwasserstoff pro 1 Epoxidäquivalent eingesetzt.

Die Herstellung der erfindungsgemässen Gemische kann in üblicher Weise durch Vermischen der Komponenten durch Handrührung oder mit Hilfe bekannter Mischaggregate, beispielsweise mittels Rührer, Kneter oder Walzen, erfolgen.

Je nach Anwendung können den erfindungsgemässen Gemischen die gebräuchlichen Zusätze beigegeben werden, wie beispielsweise Füllstoffe, Pigmente, Farbstoffe, Verlaufmittel oder Plastifizierungsmittel.

Die erfindungsgemässen Gemische zeichnen sich vorteilhaft durch eine vergleichsweise geringere Carbonatisierungstendenz auf, dass heisst, in der Kälte und bei hoher Luftfeuchtigkeit tritt in den Gemischen keine Trübung durch Aufnahme von CO₂ aus der Luft und Kristallbildung nach Umsetzung des CO₂ mit den Polyaminen auf.

Die Härtung der erfindungsgemässen Gemische kann in an sich bekannter Weise ein-oder mehrstufig vorgenommen werden. Die Härtung erfolgt im allgemeinen bei Raumtemperatur oder unterhalb der Raumtemperatur oder durch Erhitzen der Gemische auf Temperaturen bis zu 120°C, insbesondere bei Temperaturen zwischen 5 und 50°C. Um eine gute Aushärtung der erfindungsgemässen Gemische bei niedrigen Temperaturen, beispielsweise zwischen 5 und 50°C zu erzielen, können die Gemische mit Härtungsbeschleunigern auf Basis von tertiären Aminen und/oder Phenolen und/oder Alkali- oder Erdalkalisalzen eingesetzt werden, wie beispielsweise 2,4,6-Tris-(dimethylaminomethyl)-phenol, Nonylphenol, Calcium- oder Magnesiumnitrat.

Ein weiterer Gegenstand der Erfindung sind auch die durch Härten der erfindungsgemässen Gemische erhaltenen Formstoffe oder Beschichtungen.

Wie eingangs erwähnt, zeichnen sich die aus den erfindungsgemässen Gemischen hergestellten Formstoffe und Beschichtungen durch eine hohe Chemikalienresistenz, insbesondere gegenüber Carbonsäuren, wie beispielsweise wässriger Essigsäure mit einem Gehalt an Essigsäure bis zu 30 Gew-%, aus.

### Herstellung der Polyaminoamide:

### Beispiel 1

97,8 g (304,1 mMol) Ancamine® X2168 (MPCA) und 47,9 g Benzylalkohol werden auf 120°C erwärmt und mit 14,0 g (76 mMol) Gallussäuremethylester versetzt. Das Gemisch wird während 2,5 Stunden (h) bei 160°C gerührt, wobei Methanol abdestilliert wird. Nach dem Abkühlen werden 155,0 g (99% der Theorie) eines Produktegemisches erhalten, das aus 30 Gew.-% Benzylalkohol und 70 Gew.-% eines Polyamingemisches besteht, das neben dem überschüssigen MPCA hauptsächlich aus den Monoamiden der Formeln 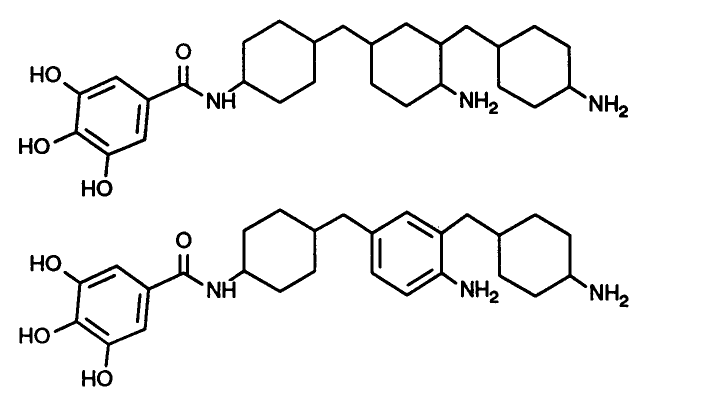 und deren Struktur- und Stereoisomeren besteht.

### Beispiel 2

34,9 g (108,6 mMol) Ancamine® X2168 (MPCA) und 20,6 g Benzylalkohol werden auf 160°C erwärmt. Nach Zugabe von 5,0 g (27,2 mMol) Gallussäuremethylester und 8,05 g (27,2 mMol) Oelsäuremethylester wird das Gemisch während 5 h bei 180°C gerührt. Nach dem Abkühlen werden 62,1 g (93% d. Th.) eines Produktegemisches erhalten, das aus 30 Gew.-% Benzylalkohol und 70 Gew.-% eines Polyamingemisches besteht, das neben dem überschüssigen MPCA hauptsächlich die Monoamide der Formeln 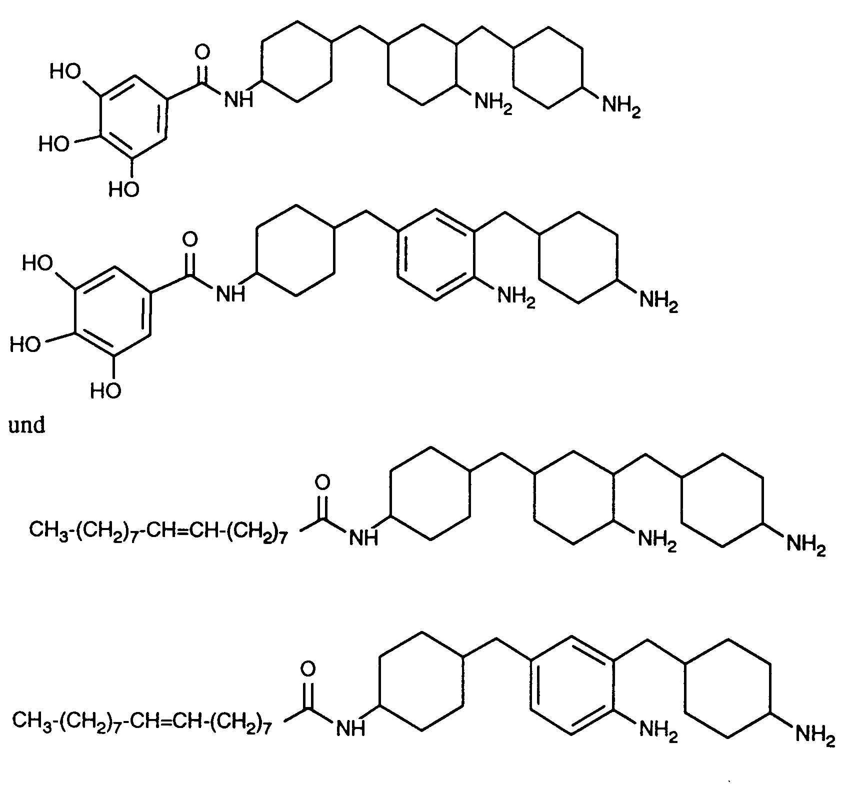 sowie deren Struktur- und Stereoisomere enthält.

### Beispiel 3

42,3 g (131,4 mMol) Ancamine® X2168 (MPCA) und 24,5 g Benzylalkohol werden auf 160°C erwärmt. Nach Zugabe von 5,0 g (32,9 mMol) 4-Hydroxybenzoesäuremethylester und 9,74 g (32,9 mMol) Oelsäuremethylester wird das Gemisch während 7,5 h bei 180°C gerührt. Nach dem Abkühlen werden 77,1 g (97% d. Th.) eines Produktegemisches erhalten, das aus 30 Gew.-% Benzylalkohol und 70 Gew.-% eines Polyamingemisches besteht, das neben dem überschüssigen MPCA hauptsächlich die Monoamide der Formeln 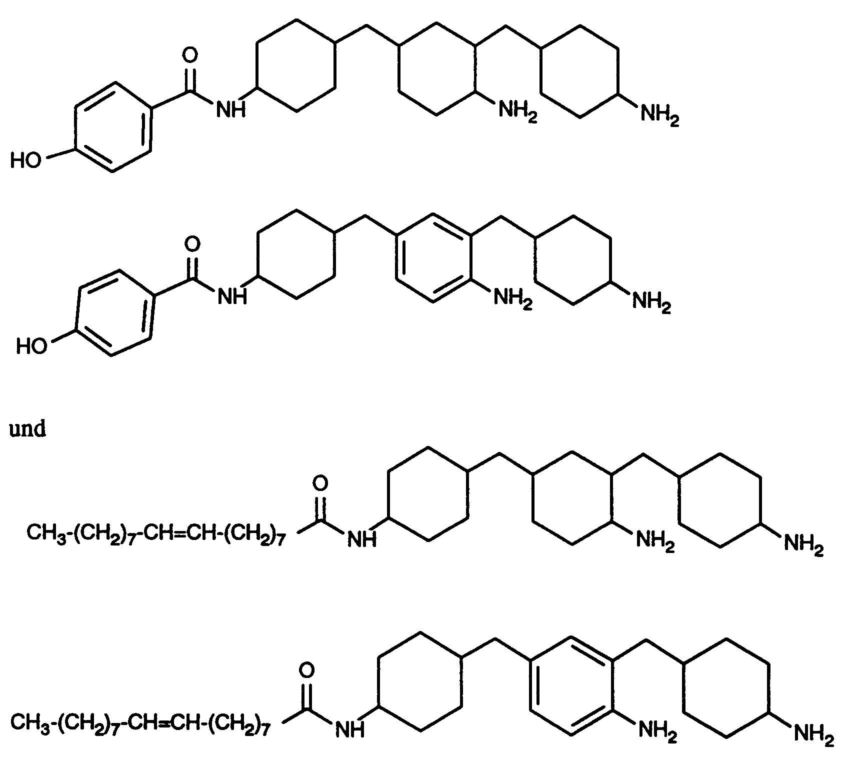 sowie deren Struktur- und Stereoisomere enthält.

### Beispiel 4

57,4 g (421,6 mMol) m-Xylylendiamin (MXDA) und 25 g (84,3 mMol) Oelsäuremethylester werden vermischt und während 5 h bei 170°C gerührt, wobei Methanol abdestilliert wird. Nach dem Abkühlen wird ein Polyamingemisch erhalten, das neben dem überschüssigen MXDA hauptsächlich das Monoamid der Formel 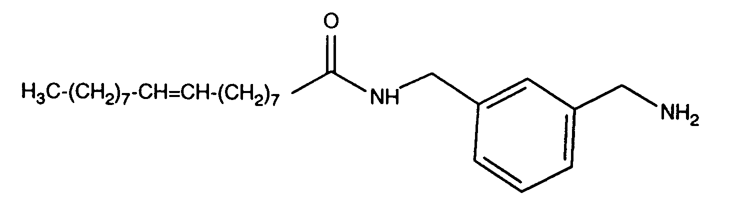 enthält.

### Beispiel 5

55,5 g (407,3 mMol) MXDA und 15,0 g (81,5 mMol) Gallussäuremethylester werden vermischt und während 3 h bei 160°C gerührt, wobei Methanol abdestilliert wird. Nach dem Abkühlen werden 66,1 g (97% d. Th.) eines grüngelben Oels erhalten, das neben dem überschüssigen MXDA hauptsächlich das Monoamid der Formel 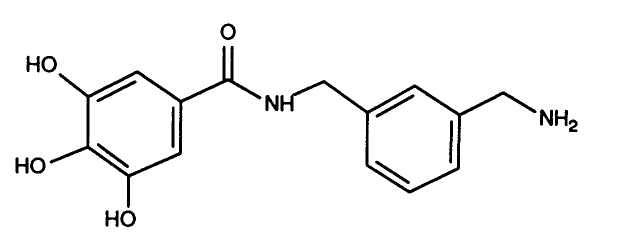 enthält.

Dieses Produkt kann wie folgt gereinigt und isoliert werden:
2,2 g des oben erhaltenen grüngelben Öles werden zweimal mit je 40 ml Dioxan ausgekocht, wobei jeweils die Dioxanphasen heiss abdekantiert werden. Durch Abkühlen der Dioxanphasen, Abdekantieren und Trocknen im Hochvakuum werden Rückstände erhalten (total 840 mg), die gemäss ¹H-NMR- und Massenspektren aus der Verbindung der oben angegebenen Formel (zu etwa 67 Mol-%) und MXDA (zu etwa 33 Mol-%) bestehen (2:1-Molekülkomplex).

### Beispiel 6

Eine Mischung von 20,0 g (58,7 mMol) Stearinsäurebutylester, 40,0 g (293,6 mMol) MXDA und 25,7 g Benzylalkohol wird während 15 h bei 200°C gerührt, wobei ein Teil des entstehenden Butanols abdestilliert wird. Nach dem Abkühlen werden 83,1 g (>100% d. Th.) eines halbfesten Produktegemisches erhalten, das aus 30 Gew.-% Benzylalkohol und 70 Gew.-% eines Polyamingemisches besteht, das neben dem überschüssigen MXDA hauptsächlich das Monoamid der Formel 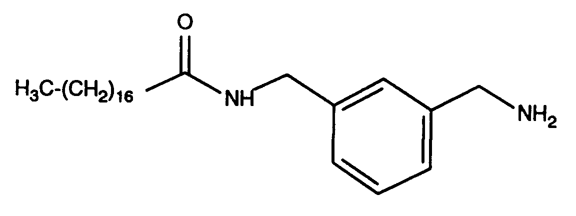 enthält.

### Beispiel 7

Eine Mischung von 18,0 g (61,6 mMol) 3-[3,5-Bis-(tert.-butyl)-4-hydroxyphenyl]-propionsäuremethylester, 42,0 g (307,8 mMol) MXDA und 25,7 g Benzylalkohol wird während 8 h bei 180°C gerührt, wobei Methanol abdestilliert wird. Nach dem Abkühlen werden 82,1 g (98% d.Th.) eines gelben Oels erhalten, das aus 30 Gew.-% Benzylalkohol und 70 Gew.-% eines Polyamingemisches besteht, das neben dem überschüssigen MXDA hauptsächlich das Monoamid der Formel 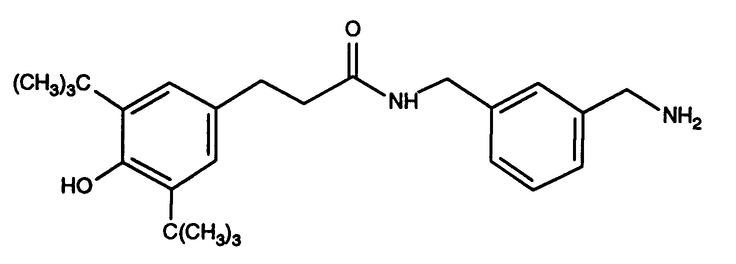 enthält.

### Beispiel 8

Ein Gemisch von 10,0 g (54,3 mMol) Gallussäuremethylester, 37,0 g (217,2 mMol) Isophorondiamin (IPD) und 20,14 g Benzylalkohol wird während 3 h bei 160°C gerührt, wobei Methanol abdestilliert wird. Nach dem Abkühlen werden 63,6 g (97% d. Th.) eines dunklen Oels erhalten, das aus 30 Gew.-% Benzylalkohol und 70 Gew.-% eines Polyamingemisches besteht, das neben dem überschüssigen IPD hauptsächlich die Monoamide der Formeln 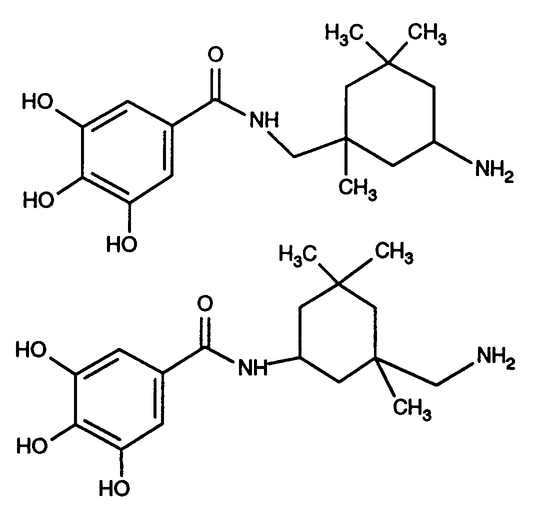 enthält.

### Beispiel 9

57,5 g (337,3 mMol) IPD und 20,0 g (67,5 mMol) Oelsäuremethylester werden vermischt und während 6 h bei 170°C gerührt, wobei Methanol abdestilliert wird. Nach dem Abkühlen werden 76,3 g (>100%) eines Produktegemisches erhalten, das neben dem überschüssigen IPD hauptsächlich aus den Monoamiden der Formeln 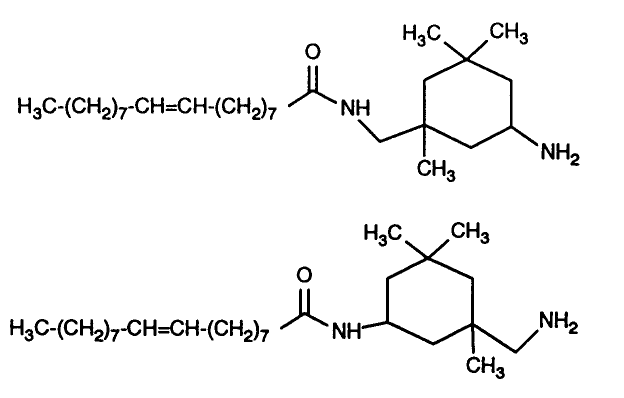 besteht.

### Beispiel 10

Ein Gemisch aus 15,0 g (44,0 mMol) Stearinsäurebutylester, 37,5 g (220,2 mMol) IPD und 22,5 g Benzylalkohol wird während 20 h bei 200°C gerührt, wobei ein Teil des entstandenen Butanols abdestilliert wird. Nach dem Abkühlen werden 71,4 g (>100% d. Th.) eines Produktegemisches erhalten, das aus 30 Gew.-% Benzylalkohol und 70 Gew.-% eines Polyamingemisches besteht, das neben dem überschüssigen IPD hauptsächlich die Produkte der Formeln 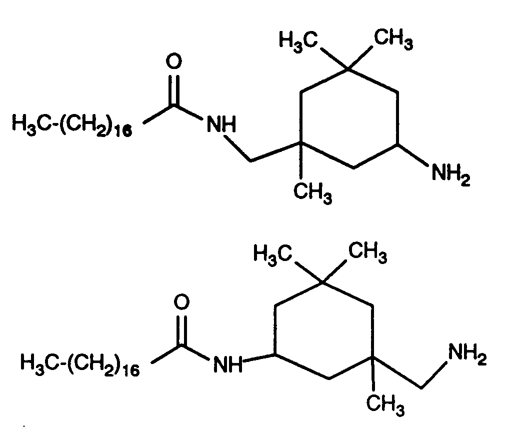 enthält.

### Beispiel 11

Ein Gemisch von 15,0 g (51,3 mMol) 3-[3,5-Bis-(tert.-butyl)-4-hydroxyphenyl-]-propionsäuremethylester, 43,7 g (256,5 mMol) IPD und 25,2 g Benzylalkohol wird während 8,5 h bei 180°C gerührt, wobei Methanol abdestilliert wird. Nach dem Abkühlen werden 79,3 g (96%) eines gelben Oels erhalten, das aus 30 Gew.-% Benzylalkohol und 70 Gew.-% eines Polyamingemisches besteht, das neben dem überschüssigen IPD hauptsächlich die Produkte der Formeln 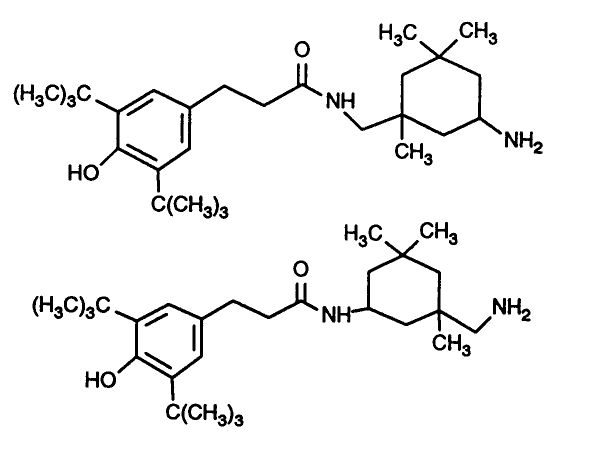 enthält.

### Beispiel 12

211,34 g (657,25 mMol) Ancamine® X2168 (MPCA) und 101,5 g Benzylalkohol werden auf 160°C erwärmt. Nach Zugabe von 25 g (164,31 mMol) 4-Hydroxybenzoesäuremethylester wird das Gemisch während 8 h bei 180°C gerührt, wobei Methanol abdestilliert wird. Nach dem Abkühlen werden 328,84 g (99%) eines Produktegemisches erhalten, das aus 30 Gew.-% Benzylalkohol und 70 Gew.-% eines Polyamingemisches besteht, das neben dem überschüssigen MPCA hauptsächlich die Monoamide der Formeln 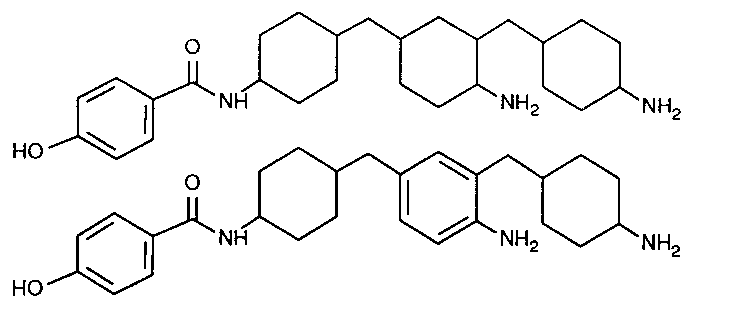 sowie deren Struktur- und Stereoisomere enthält.

### Anwendunnsbeispiele

### Beispiel I

2,5 g des Härters gemäss Beispiel 1 werden mit 5,0 g Bisphenol A-diglycidylether mit einem Epoxidgehalt von 5,25 - 5,40 Äquivalenten/kg und einer Viskosität von 10000 - 12000 mPa·s gründlich vermischt. Das klare Gemisch wird mittels Pinsel auf eine sandgestrahlte, entfettete Stahlplatte oder mittels Rakel (0,20 mm) auf eine Glasplatte aufgetragen. Nach wenigen Stunden bei 20°C entstehen klare, harte Schichten, die auf der Glasplatte nach einem Tag bei 20°C eine Persoz-Härte von 266 s aufweisen, gemessen mit einem Pendeldämpfungsprüfer TY 5853 (BYK-Chemie) nach Persoz. Nach einer Durchhärtung während 7 Tagen bei 20°C ist die Persoz-Härte auf 326 s gestiegen. Die Schicht auf der Stahlplatte widersteht dann einer Belastung durch Benetzung mit 10%iger wässriger Essigsäure während mindestens drei Monaten, ohne Löcher zu bilden oder sich abzulösen.
Das oben beschriebene härtbare Gemisch härtet auch bei 20°C/ 100% relativer Luftfeuchtigkeit sowie bei 5°C/45% relativer Luftfeuchtigkeit befriedigend aus, wobei keine oder nur eine sehr geringe Oberflächentrübung auftritt.

### Beispiel II

2,0 g des Härters gemäss Beispiel 1 und 0,5 g des Härters gemäss Beispiel 2 werden zusammen mit 5,0 g Bisphenol A-diglycidylether mit einem Epoxidgehalt von 5,25 - 5,40 Äquivalenten/kg und einer Viskosität von 10000 - 12000 mPa·s und 0,5 g 4-Nonylphenol gründlich vermischt. Das klare Gemisch wird mittels Pinsel auf eine sandgestrahlte, entfettete Stahlplatte oder mittels Rakel (0,20 mm) auf eine Glasplatte aufgetragen. Nach wenigen Stunden bei 20°C entstehen klare, harte Schichten, die auf der Glasplatte nach einem Tag bei 20°C eine Persoz-Härte von 165 s aufweisen, gemessen mit dem Pendeldämpfungsprüfer TY 5853 (BYK-Chemie) nach Persoz. Nach einer Durchhärtung während 7 Tagen bei 20°C ist die Persoz-Härte auf 268 s gestiegen. Die Schicht auf der Stahlplatte widersteht dann einer Belastung durch Benetzung mit 10%iger wässriger Essigsäure während mindestens drei Monaten, ohne Löcher zu bilden oder sich abzulösen. Das oben beschriebene härtbare Gemisch härtet auch bei 20°C/100% relativer Luftfeuchtigkeit sowie bei 5°C/45% relativer Luftfeuchtigkeit befriedigend aus, wobei keine oder nur eine sehr geringe Oberflächentrübung auftritt.

Wird in der oben angegebenen Formulierung das 4-Nonylphenol weggelassen, werden nach 1 Tag bei 20°C eine Persoz-Härte von 233 s, nach 7 Tagen bei 20°C eine Persoz-Härte von 297 s erreicht. Die sonstigen erwähnten Eigenschaften, insbesondere die Resistenz gegen 10%ige wässrige Essigsäure, sind sehr ähnlich.

### Beispiel III

2,5 g des Härters gemäss Beispiel 3 werden zusammen mit 3,8 g Bisphenol A-diglycidylether mit einem Epoxidgehalt von 5,25 - 5,40 Äquivalenten/kg und einer Viskosität von 10000 - 12000 mPa·s und 1,0 g 4-Nonylphenol gründlich vermischt. Das klare Gemisch wird mittels Pinsel auf eine sandgestrahlte, entfettete Stahlplatte oder mittels Rakel (0,20 mm) auf eine Glasplatte aufgetragen. Nach wenigen Stunden bei 20°C entstehen klare, harte Schichten, die auf der Glasplatte nach einem Tag bei 20°C eine Persoz-Härte von 52 s aufweisen, gemessen mit dem Pendeldämpfungsprüfer TY 5853 (BYK-Chemie) nach Persoz. Nach 7 Tagen Durchhärtung bei 20°C ist die Persoz-Härte auf 204 s gestiegen. Die Schicht auf der Stahlplatte widersteht dann einer Belastung durch Benetzung mit 10%iger wässriger Essigsäure während mindestens drei Monaten, ohne Löcher zu bilden oder sich abzulösen.
Das oben beschriebene härtbare Gemisch härtet auch bei 20°C/100% relativer Luftfeuchtigkeit sowie bei 5°C/45% relativer Luftfeuchtigkeit befriedigend aus, wobei keine oder nur eine sehr geringe Oberflächentrübung auftritt.
Wird in der obigen Formulierung das 4-Nonylphenol durch 0,5 g Benzylalkohol ersetzt, werden nach 1 Tag bei 20°C eine Persoz-Härte von 32 s, nach 7 Tagen von 255 s erreicht. Die sonstigen erwähnten Eigenschaften, insbesondere die Resistenz gegen 10%ige wässrige Essigsäure, sind sehr ähnlich.

### Beispiel IV

2,5 g des Härters gemäss Beispiel 12 werden zusammen mit 5,05 g Bisphenol A-diglycidylether mit einem Epoxidgehalt von 5,25 - 5,40 Äquivalenten/kg und einer Viskosität von 10000 - 12000 mPa·s und 2,0 g 4-Nonylphenol gründlich vermischt. Das klare Gemisch wird mittels Pinsel auf eine sandgestrahlte, entfettete Stahlplatte oder mittels Rakel (0,20 mm) auf eine Glasplatte aufgetragen. Nach wenigen Stunden bei 20°C entstehen klare, harte Schichten, die auf der Glasplatte nach einem Tag bei 20°C eine Persoz-Härte von 86 s aufweisen, gemessen mit dem Pendeldämpfungsprüfer TY 5853 (BYK-Chemie) nach Persoz. Nach 7 Tagen Durchhärtung bei 20°C ist die Persoz-Härte auf 197 s gestiegen. Die Schicht auf der Stahlplatte widersteht dann einer Belastung durch Benetzung mit 10%iger wässriger Essigsäure während mindestens drei Monaten, ohne Löcher zu bilden oder sich abzulösen.
Das oben beschriebene härtbare Gemisch härtet auch bei 20°C/100% relativer Luftfeuchtigkeit sowie bei 5°C/45% relativer Luftfeuchtigkeit befriedigend aus, wobei keine oder nur eine sehr geringe Oberflächentrübung auftritt.
Wird in der obigen Formulierung das 4-Nonylphenol durch 0,5 g Benzylalkohol ersetzt, werden nach 1 Tag bei 20°C eine Persoz-Härte von 83 s, nach 7 Tagen von 292 s erreicht. Die sonstigen erwähnten Eigenschaften, insbesondere die Resistenz gegen 10%ige wässrige Essigsäure, sind sehr ähnlich.

## Patentansprüche

1. Monocarbonsäureamide der Formel I 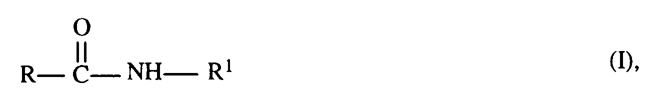 worin R für einen Rest der Formeln 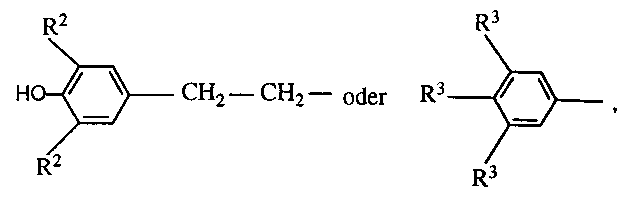 C₉-C₁₉-Alkyl, C₉-C₁₉-Alkenyl oder ein C₉-C₁₉-Alkdienyl steht,
wobei jedes R² unabhängig voneinander je ein Wasserstoffatom oder ein C₁-C₄-Alkyl bedeutet, jedes R³ unabhängig voneinander je ein Wasserstoffatom oder eine Hydroxylgruppe bedeutet und mindestens ein R³ für eine Hydroxylgruppe steht, und R¹ für einen Rest der Formeln 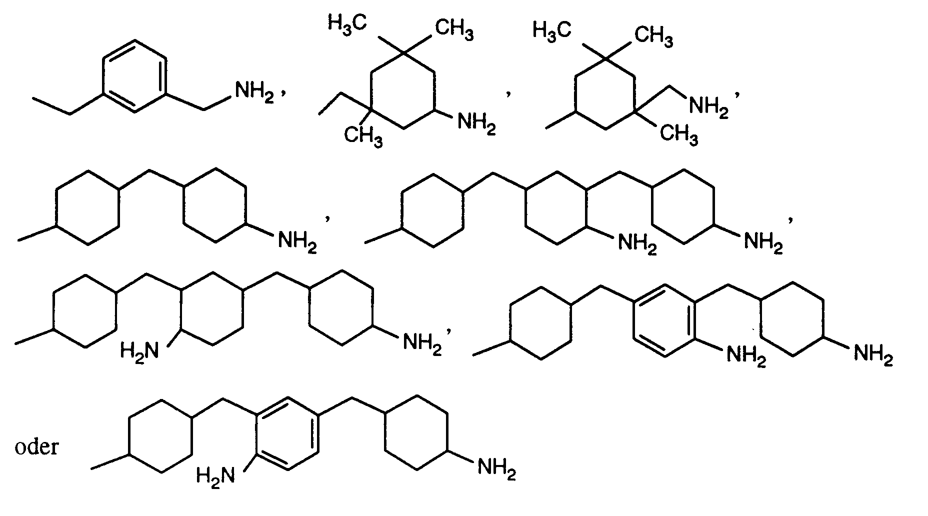 steht.

2. Monocarbonsäureamide gemäss Anspruch 1, dadurch gekennzeichnet, dass R in der Formel I für einen Rest der Formeln 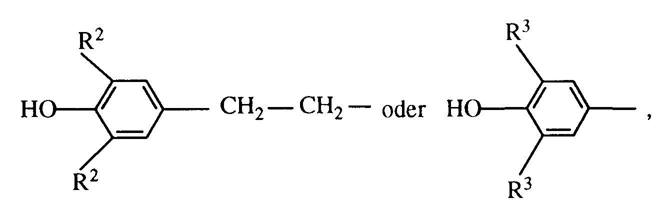 oder ein C₁₅-C₁₇-Alkyl oder C₁₅-C₁₇-Alkenyl steht, wobei jedes R² ein tert.-Butyl bedeutet, und jedes R³ je ein Wasserstoffatom oder je eine Hydroxylgruppe bedeutet.

3. Monocarbonsäureamide gemäss Anspruch 1, dadurch gekennzeichnet, dass R in der Formel I für den Rest der Formel 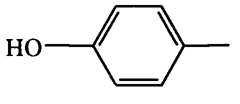 steht.

4. Monocarbonsäureamide gemäss Anspruch 1, dadurch gekennzeichnet, dass R¹ in der Formel I für einen Rest der Formeln 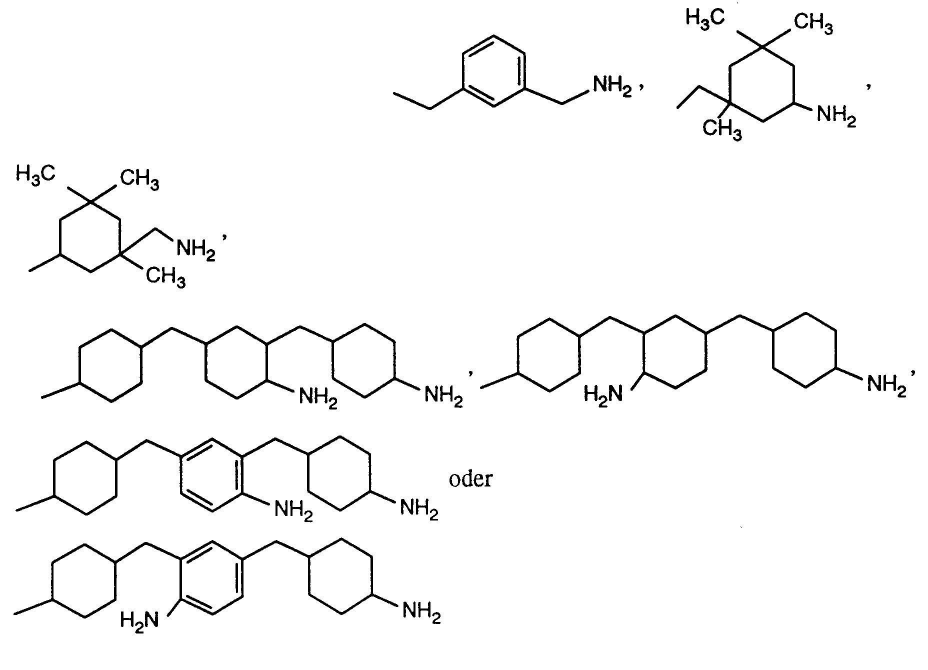 steht.

5. Monocarbonsäureamide gemäss Anspruch 1 der Formeln 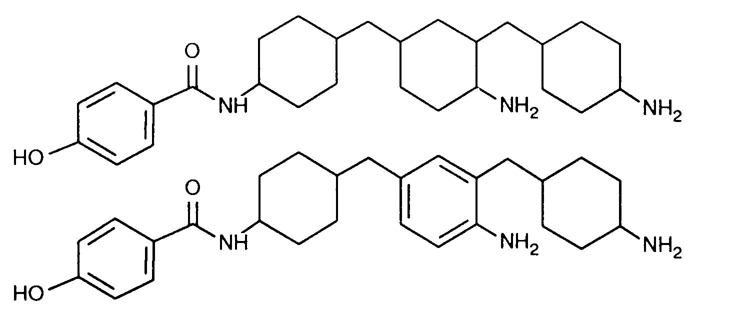 sowie deren Struktur- und Stereoisomere.

6. Härtungsmittel für Epoxidharze, dadurch gekennzeichnet, dass es mindestens eine Verbindung der Formel I gemäss Anspruch 1 enthält.

7. Härtungsmittel für Epoxidharze gemäss Anspruch 6, das erhalten wird, indem man eine Monocarbonsäure oder deren Ester der Formel II
R―COOR⁴ (II),
worin R die gleiche Bedeutung wie in Formel I hat und R⁴ ein Wasserstoffatom, Alkyl oder Phenyl bedeutet, mit einem Di- oder Triamin der Formel III
H₂N―R¹ (III),
worin R¹ die gleiche Bedeutung wie in Formel I hat, bei erhöhter Temperatur umsetzt, wobei man auf 1 Mol einer Verbindung der Formel II 2 bis 20 Mole einer Verbindung der Formel III einsetzt.

8. Härtbares Gemisch, enthaltend
(a) ein Epoxidharz mit mehr als einer 1,2-Epoxidgruppe im Molekül und
(b) eine Verbindung der Formel I gemäss Anspruch 1.

9. Die durch Härten der Gemische gemäss Anspruch 8 erhaltenen Formstoffe oder Beschichtungen.

## Claims

1. A monocarboxamide of the formula I 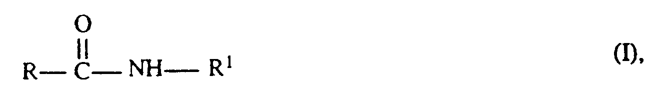 in which R is a radical of the formula 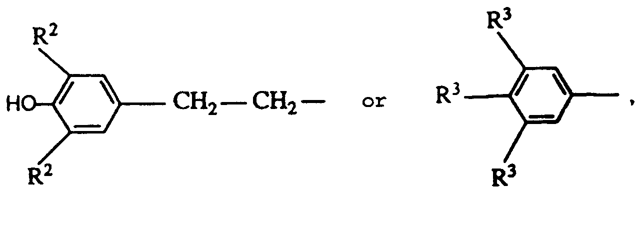 C₉-C₁₉alkyl, C₉-C₁₉alkenyl or a C₉-C₁₉alkdienyl,
where each R² independently of the other is a hydrogen atom or a C₁-C₄alkyl, each R³ independently of the others is a hydrogen atom or a hydroxyl group and at least one R³ is a hydroxyl group, and R¹ is a radical of the formula

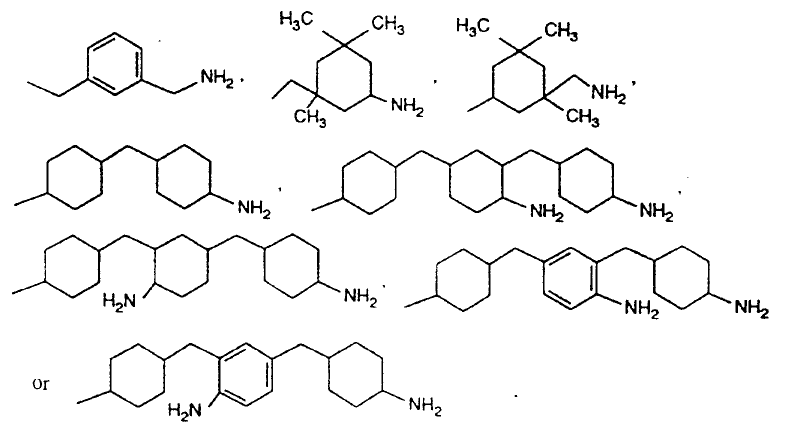

2. A monocarboxamide according to claim 1, wherein R in the formula I is a radical of the formula 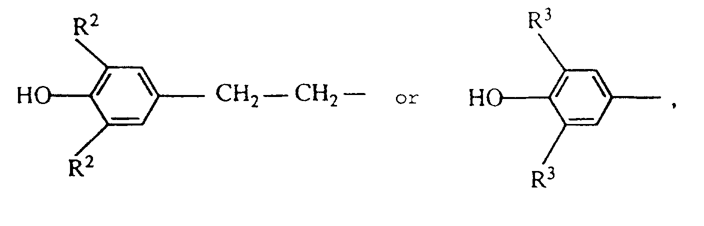 a C₁₅-C₁₇alkyl or C₁₅-C₁₇alkenyl, in which each R² is a tert-butyl and each R³ is a hydrogen atom or a hydroxyl group.

3. A monocarboxamide according to claim 1, wherein R in the formula I is the radical of the formula

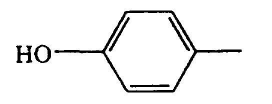

4. A monocarboxamide according to claim 1, wherein R¹ in the formula I is a radical of the formula

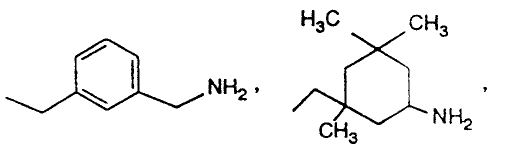

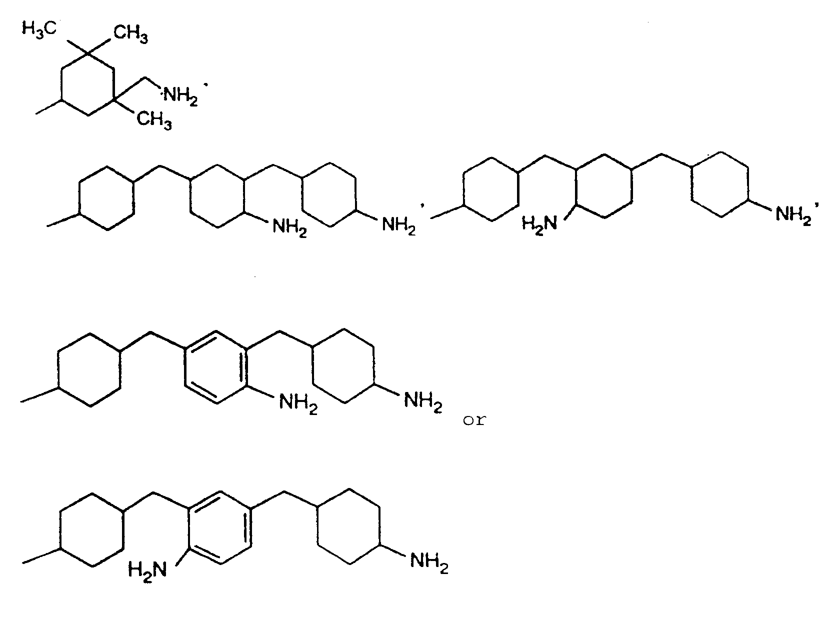

5. A monocarboxamide according to claim 1 of one of the formulae 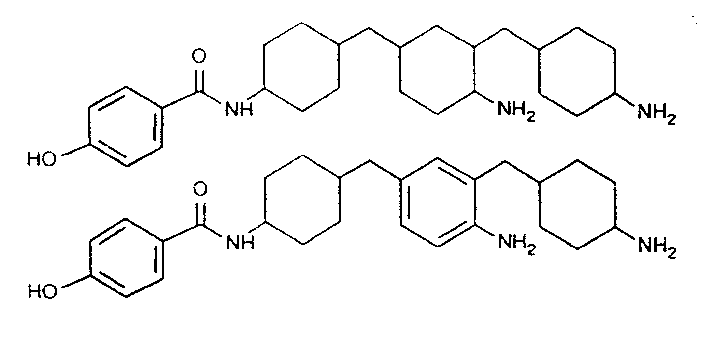 or a structural isomer or stereoisomer thereof.

6. A curing agent for expoxy resins, which comprises at least one compound of the formula I according to claim 1.

7. A curing agent for epoxy resins according to claim 6, which is obtained by reacting a monocarboxylic acid or an ester thereof, of the formula II
R-COOR⁴ (II)
in which R has the same meaning as in formula I and R⁴ is a hydrogen atom, alkyl or phenyl, with a di- or triamine of the formula III
H₂N-R¹ (III)
in which R¹ has the meaning as in formula I, at an elevated temperature, employing from 2 to 20 mol of a compound of the formula III per mole of a compound of the formula II.

8. A curable mixture comprising
(a) an epoxy resin having more than one 1,2-epoxide group in the molecule and
(b) a compound of the formula I according to claim 1.

9. A shaped article or coating obtained by curing a mixture according to claim 8.

## Revendications

1. Monocarboxamides de formule I 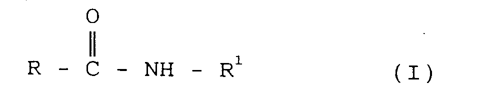 dans laquelle R est un radical ayant les formules 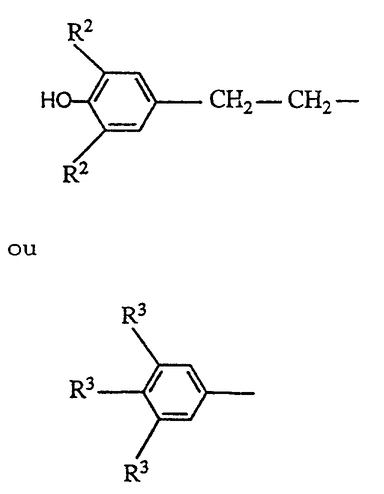 un groupe alkyle en C₉-C₁₉, alcényle en C₉-C₁₉ ou alcadiényle en C₉-C₁₉,
où chaque radical R² est, indépendamment de l'autre, un atome d'hydrogène ou un groupe alkyle en C₁-C₄, chaque radical R³ est indépendamment des autres un atome d'hydrogène ou un groupe hydroxyle, et au moins un radical R³ est un groupe hydroxyle, et R¹ est un radical ayant les formules suivantes :

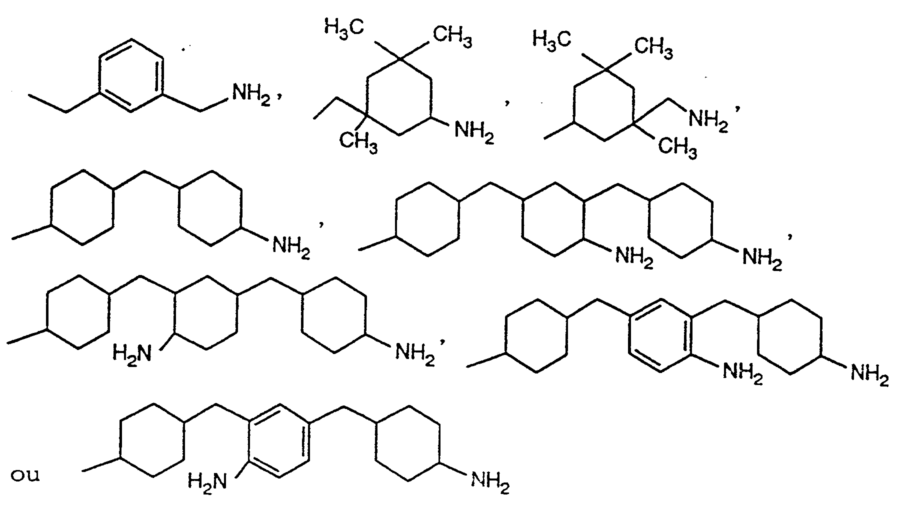

2. Monocarboxamides selon la revendication 1, caractérisés en ce que R, dans la formule I, représente un radical ayant les formules 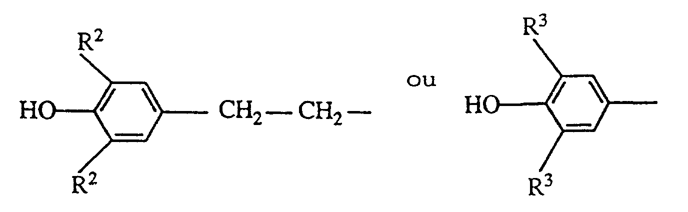 un groupe alkyle en C₁₅-C₁₇ ou alcényle en C₁₅-C₁₇, chaque radical R² étant un groupe tert-butyle et chaque radical R³ étant un atome d'hydrogène ou un groupe hydroxyle.

3. Monocarboxamides selon la revendication 1, caractérisés en ce que R, dans la formule I, représente le radical de formule

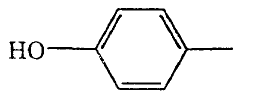

4. Monocarboxamides selon la revendication 1, caractérisés en ce que R¹ dans la formule I, représente un radical ayant les formules

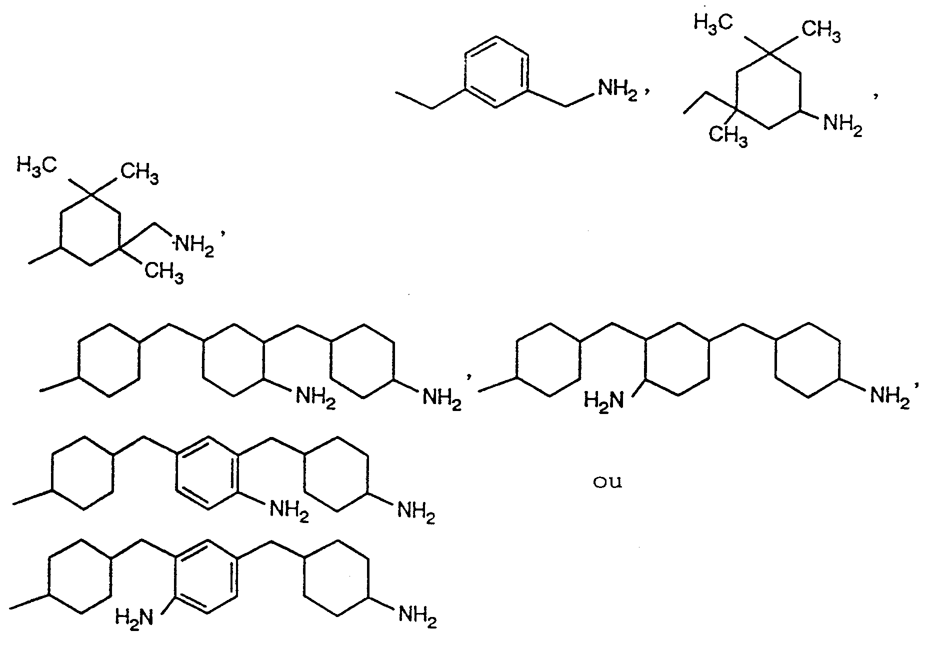

5. Monocarboxamides selon la revendication 1, ayant les formules 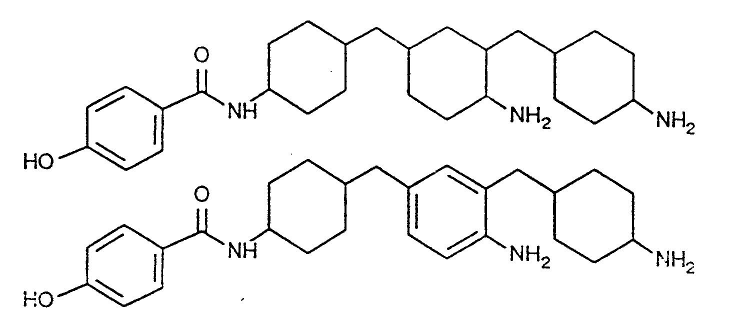 ainsi que leurs isomères de structure et stéréoisomères.

6. Agent de durcissement pour résines époxydes, caractérisé en ce qu'il contient au moins un composé de formule I selon la revendication 1.

7. Agent de durcissement pour résines époxydes selon la revendication 6, que l'on obtient en faisant réagir un acide monocarboxylique ou ses esters de formule II
R - COOR⁴ (II)
dans laquelle R a les mêmes significations que dans la formule I et R⁴ est un atome d'hydrogène ou un groupe alkyle ou phényle, avec une di- ou une triamine de formule III
H₂N - R¹ (III)
dans laquelle R¹ a les mêmes significations que dans la formule I, à haute température, où on utilise par mole d'un composé de formule II une quantité de 2 à 20 moles d'un composé de formule III.

8. Mélange durcissable contenant
(a) une résine époxyde contenant dans sa molécule plus d'un groupe 1,2-époxy, et
(b) un composé de formule I selon la revendication 1.

9. Mélange à mouler ou revêtements obtenus par durcissement des mélanges selon la revendication 8.
